# EUROPÄISCHE PATENTSCHRIFT

(11) **EP 0 799 646 B1**
(45) Veröffentlichungstag und Bekanntmachung des Hinweises auf die Patenterteilung: **18.09.2002**
(21) Anmeldenummer: 97105075.2
(22) Anmeldetag: 26.03.1997
(51) Int. Cl.: B05B 11/00, B05B 11/06, A61M 15/00

(54) **Dosiereinrichtung für strömungsfähige Medien wie Pulver/Luft-Dispersionen**
Dosing device for flowing media such as air-powder dispersion
Dispositif de dosage pour une substance apte à s'écouler comme une dispersion air-poudre

(30) Priorität: 02.04.1996 DE 19613185
(43) Veröffentlichungstag der Anmeldung: 08.10.1997
(73) Patentinhaber: ING. ERICH PFEIFFER GMBH, 78315 Radolfzell (DE)
(72) Erfinder: Fuchs, Karl-Heinz, 78315 Radolfzell (DE)
(74) Vertreter: Patentanwälte Ruff, Wilhelm, Beier, Dauster & Partner

(56) Entgegenhaltungen:
- EP-A- 0 473 965
- GB-A- 674 355
- US-A- 4 261 488

## Beschreibung

Die Erfindung betrifft eine Dosiereinrichtung für strömungsfähige Medien, insbesondere in Austragvorrichtungen für pharmazeutische oder kosmetische Produkte, wie Pulver/Luft-Dispersionen, wobei das Medium einen Austragskanal durchströmt der einen Verschluß aufweist.

Bei der Verabreichung von Pharmazeutika ist stets die Dosierung ein wesentliches Problem. Dabei ist die Wirkung eines Produktes oft davon abhängig oder wird zumindest verbessert, wenn man es in Form von Aerosolen verabreicht, d.h. bei flüssigen Produkten in Luft zerstäubt oder bei festen Produkten in Form eines Festkörper-Aerosols bzw. einer entsprechenden Dispersion eines Pulvers in Luft. Solche Dispersionen bzw. Aerosole kann man durch Ausbringen mit Pumpen erzeugen, wobei es bei flüssigen Produkten möglich ist, die Flüssigkeit unmittelbar in fein zerstäubter Form in die Umgebungsluft einzubringen, d.h. die Luft nicht als Zerstäubungsmittel einzusetzen. Bei Pulvern ist dies schwieriger. Hier ist auch die Dosierung besonders problematisch.

Es sind schon Pulverzerstäubungseinrichtungen bekannt geworden (DE 41 28 295 A), bei denen der Pulvervorrat in Form kleiner Kapseln vorliegt, die von einem Luftstrom "leergeblasen" werden, um so eine Dosierung einer Pulvermenge zu erreichen. Diese Vorrichtungen sind aber kompliziert und erfordern eine aufwendige Verpackung der einzelnen Pulverkapseln. Es ist auch schwer, die Kapseln bei der Benutzung gleichmäßig zu entleeren, so daß auch dort die ausgegebene Menge in weiten Grenzen schwankt.

Die GB 674 355 (vgl. Oberbegriff des Anspruchs 1, bzw. 2) zeigt einen Quetschbehälter mit einer Ausgabevorrichtung, die an einem in den Behälter hineinragenden Rohr eine Meßkammer aufweist, in der eine Kugel zwischen einer auf dem Behälterboden aufliegenden Ruhelage in eine Lage am anderen Ende des Meßbehälters bewegbar ist. Die Kugel wird in der Meßkammer gleitend geführt.

### AUFGABE UND LÖSUNG

Aufgabe der Erfindung ist es, eine Dosiereinrichtung der eingangs erwähnten Art zu schaffen, mit der mit einfachen Mitteln und möglichst unabhängig von der Art der Erzeugung bzw. Aufbereitung des auszugebenden Mediums eine Dosierung möglich ist.

Diese Aufgabe wird erfindungsgemäß durch den Anspruch 1 oder den Anspruch 2 gelöst. Dadurch, daß der Verschluß im Austragskanal unter der Wirkung der Strömung von einer Startposition zeitverzögert in eine Schließposition bewegbar ist, erzeugt der Ausgabevorgang, nämlich die Strömung selbst das Ende der Dosierung.

Sein Anfang ist ohnehin durch den Beginn der Austragung festgelegt.

Der Verschluß weist einen Verschlußkörper auf, der mit einem Ventilsitz zusammenarbeitet und beispielsweise die Form einer Kugel haben kann. Dieser ist über eine die Zeitverzögerung bewirkende Wegstrecke bewegbar.

Die auf ihn einwirkende Kraft, die die Bewegung verursacht, kann beispielsweise der auf die Kugel wirkende Strömungswiderstand des strömenden Mediums sein. In einem solchen Falle kann der Verschlußkörper in einem ihm gegenüber erweiterten Austragskanal angeordnet sein. Es ist auch möglich, daß er in seiner Startposition einen Ruheverschluß für den Austragskanal bewirkt, indem er auf einem zweiten Ventilsitz aufsitzt.

Hierzu sei bemerkt, daß es bei Zerstäubern bereits Einrichtungen gibt, bei denen ein Ventilkörper mit zwei einander gegenüberliegenden Ventilsitzen wahlweise zusammenarbeitet. Eine solche Ausführung ist in der EP 0 201 701 B der Anmelderin gezeigt. Dort ist dieses in beiden Richtungen wirkende Ventil als Ansaugventil vorgesehen, das auch bei Überkopflage funktionieren soll.

Auch bei der Erfindung kann die Schwerkraft eine Rolle spielen. Dies ist die einfachste Möglichkeit, um die Kraft zu schaffen, die der durch die Strömung erzeugten Wirkung entgegen arbeitet. Auch die Umkehrung der Schwerkraft, die Auftriebskraft des Ventilkörpers, könnte bei entsprechenden Voraussetzungen eingesetzt werden. Es könnten jedoch auch Federn oder andere Kräfte, beispielsweise auch Magnetkraft, als der Strömungswirkung entgegen gerichtete Kräfte verwendet werden. Bei der Dosierung wird durch die Wirkung der Strömung der Ventilkörper in Bewegung gesetzt, und zwar vorzugsweise mit einer von der Geschwindigkeit der Strömung abhängigen Kraftwirkung. Dies führt dazu, daß auch bei unterschiedlichen Strömungsgeschwindigkeiten die Dosierung recht gleichmäßig erfolgt, d.h. bei geringeren Strömungsgeschwindigkeiten wird es länger dauern, bis der Verschluß die Dosierung beendet, während bei höheren Strömungsgeschwindigkeiten die Dosierung früher beendet wird. Auf jeden Fall wird wirksam vermieden, daß eine unkontrollierte Menge, die zu einer erheblichen Überdosierung führen könnte, ausgegeben wird.

Es hat sich auch gezeigt, daß infolge der nach Einleitung der Bewegung entstehenden Massenträgheitskräfte bei entsprechender Auslegung die Einstellung eines Gleichgewichtes, d.h. das Halten des Verschlußkörpers in einer Lage, in der er den Verschluß nicht erreicht, fast ausgeschlossen ist. Wenn man die Einrichtung so bemißt, daß in jedem Falle, in dem eine die Pulver/Luft-Dispersion erzeugende Strömungsgeschwindigkeit vorliegt, der Verschlußkörper in Bewegung gesetzt wird, dann führt er diese Bewegung auch bis zum Verschluß zu Ende.

Die Verschlußcharakteristik kann durch verschiedene Faktoren geändert werden. So kann Form und Absolutgewicht sowie spezifisches Gewicht der Verschlußkörper die Charakteristik ebenso beeinflussen, wie ihr Verhältnis zu dem Kanal, in dem sie laufen. So kann beispielsweise eine Durchmesseränderung über die Länge der Bewegungsstrecke dazu führen, eine gewünschte Dosiercharakteristik zu erzielen. So ist es beispielsweise möglich, durch eine relativ hohe Kraft (großes Gewicht oder zusätzliche Belastung) den Ruheverschluß so lange aufrechtzuerhalten, bis ein zur Erzeugung einer ausreichenden Strömung geeigneter Druck aufgebaut ist, danach einen relativ großen Umströmungsquerschnitt des Ventilkörpers vorzusehen, um eine ausreichende Verzögerungszeit zu erreichen und danach diesen Umströmungsquerschnitt wieder zu verkleinern, um einerseits die Überführung in die Verschlußlage unter allen Umständen sicherzustellen und andererseits vor dem Dosierende eine gewisse Drosselung herbeizuführen, so daß das Dosierende nicht zu schlagartig erfolgt. Dies gilt insbesondere in dem Fall, wenn, wie es bevorzugt ist, die Dosiereinrichtung bei einem Inhalator eingesetzt ist, bei dem die Strömung durch orale Ansaugung über ein Mundstück erfolgt.

Bei einer solchen Ausführung kann das auszugebende Pulver in einem Behälter vorhanden sein und sich beim Ansaugen selbst von der Oberfläche hochsaugen und in der ausreichenden Menge in der Luft anreichern. In diesem Falle muß der Behälter eine Belüftung haben, damit Luft nachströmen kann.

Bei einer bevorzugten Ausführung der Erfindung kann für das Austragen einer Pulver/Luft-Dispersion über eine Austrageinrichtung aus einem Pulver enthaltenden Behälter, in dem diese Dispersion erzeugt wird, eine in den Behälter führende Belüftungsöffnung in oder auf den Pulvervorrat in dem Behälter gerichtet sein. Die infolge des Austrags nachgesaugte Luft kann eine Aufwirbelung des Pulvers, d.h. die Erzeugung dieser Dispersion, bewirken. Man braucht also lediglich von dem Belüftungskanal ein Röhrchen auf die Pulveroberfläche oder unter diese führen, so daß die nachgesaugte Luft eine gezielte Aufwirbelung erzeugt.

Die Wirkung, die die Strömung auf den Verschlußkörper ausübt, kann auch auf andere Weise erzeugt werden, beispielsweise durch einen von der Strömung erzeugten Unterdruck in einem Seitenkanal, wodurch ein darin liegender Verschlußkörper in die Verschließlage gesaugt wird.

Die Ausführung, in der der Verschlußkörper auf einen zweiten Ventilsitz einwirkt, der den Behälter in Ruheposition verschließt, sorgt dafür, daß das meist hydrophile Pulver von der Atmosphäre weitgehend abgeschlossen ist. Dies kann auch dadurch verbessert werden, daß beispielsweise eine Kappe über die Austragseinrichtung gesetzt wird, die die Belüftungsöffnung ebenso verschließt wie den Austragskanal und die z.B. mit einem Stift den Verschlußkörper in der Ruhe-Verschlußposition hält.

Diese und weitere bevorzugte Ausführungen gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung verwirklicht sein können.

Die Unterteilung der Anmeldung in einzelne Abschnitte sowie Zwischen-Überschriften beschränken die unter diesen gemachten Aussagen nicht in ihrer Allgemeingültigkeit.

### KURZBESCHREIBUNG DER ZEICHNUNGEN

Ein Ausführungsbeispiel der Erfindung ist in den Zeichnungen dargestellt und wird im folgenden näher erläutert. In den Zeichnungen zeigen:
- Fig. 1: einen vertikalen Schnitt durch einen Pulver-Inhalator mit einer Dosiereinrichtung nach der Erfindung in der Startposition
- Fig. 2: eine Darstellung der Dosiereinrichtung nach Fig. 1 in ihrer Schließposition,
- Fig. 3: ein Detail einer abgewandelten Ausführungsform,
- Fig. 4: eine nach einem Saugprinzip arbeitende Ausführung in gleicher Darstellungsweise, sowie
- Fig. 5 bis 8: vertikale Teilschnitte durch eine Dosiereinrichtung mit Vordosierung in vier Funktionsstellungen.

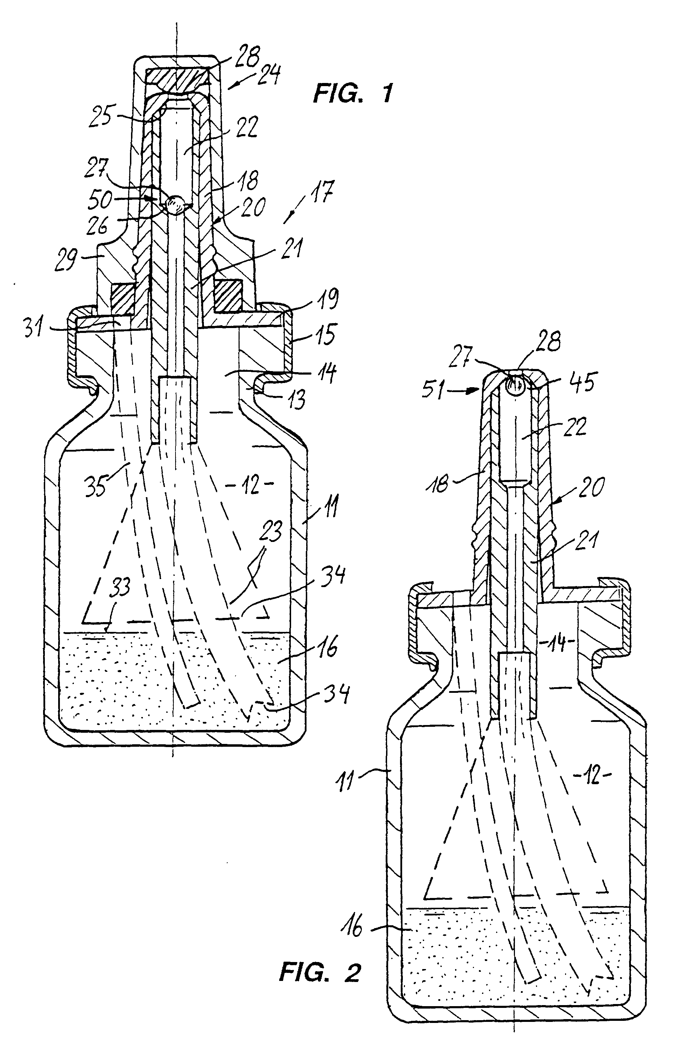

### BESCHREIBUNG DER AUSFÜHRUNGSBEISPIELE FIG. 1 BIS 4

Fig. 1 und 2 zeigen eine Dosiereinrichtung 17, die mittels eines Krimpverschlusses 15, der einen Flansch 19 des Gehäuses 20 der Dosiereinrichtung übergreift, auf einem Behälter 11 angebracht ist, in dessen Innenraum 12 ein Vorrat eines Pulvers 16 enthalten ist. Der Behälter kann eine normale Flasche mit einem Hals 13 und einer Öffnung 14 sein, es sind jedoch auch andere Behälterformen möglich.

Die Dosiereinrichtung 17 hat anschließend an den Flansch 19 ein Mundstück 18, das an eine eine Saugwirkung ausübende Körperöffnung, beispielsweise ein Nasenloch oder den Mund angepaßt ist. Es weist an seinem oberen Ende eine Mündung 28 auf, an der ein Austragskanal 22 endet. Dieser ist in einem Einsatz 21 vorgesehen, der in das Gehäuse 20 eingepresst ist.

Das Gehäuse bildet an seinem oberen Ende nahe der Mündung 28 einen Ventilsitz 25 eines Verschlusses 24. Von dort an hat der Austragskanal über eine größere Strecke, die um ein vielfaches axial länger ist als ein darin angeordneter Ventilkörper 27 in Form einer Kugel, einen Durchmesser, der größer ist als diese Kugel, so daß sich ein den Ventilkörper umgebender Umströmungsquerschnitt 45 bildet. An dem dem Ventilsitz 25 gegenüberliegenden Ende des erweiterten Austragskanales 22 ist ein weiterer Ventilsitz 26 vorgesehen, auf dem Ventilkörper 27 in seiner Ruheposition aufliegt und den Austragskanal abdichtet. Von diesem Ventilsitz aus in den Behälter hinein erstreckt sich ein Saugrohr 23, das, wie strichliert angedeutet, entweder in das Pulver hinein bis nahe dem Boden des Behälters reicht oder z.B. in Form eines Trichters im Bereich der Pulveroberfläche 33 eine Ansaugöffnung 34 hat.

Über das Gehäuse 20 und insbesondere das Mundstück 18 der Dosiereinrichtung greift eine Kappe 19, die auf ein Gewinde des Mundstückes geschraubt ist und sowohl die Mündung 28 abschließt als auch einen in den Flansch 19 mündenden Belüftungskanal 31 zwischen der Atmosphäre und dem Behälter. Der Belüftungskanal kann als eine einfache Öffnung in dem Flansch vorgesehen sein, es ist jedoch auch möglich, dort ein Rohr 35 anzuschließen. Dieses strichpunktiert dargestellte Belüftungsrohr 35 führt unter die Pulveroberfläche und hat dort seine Mündung.

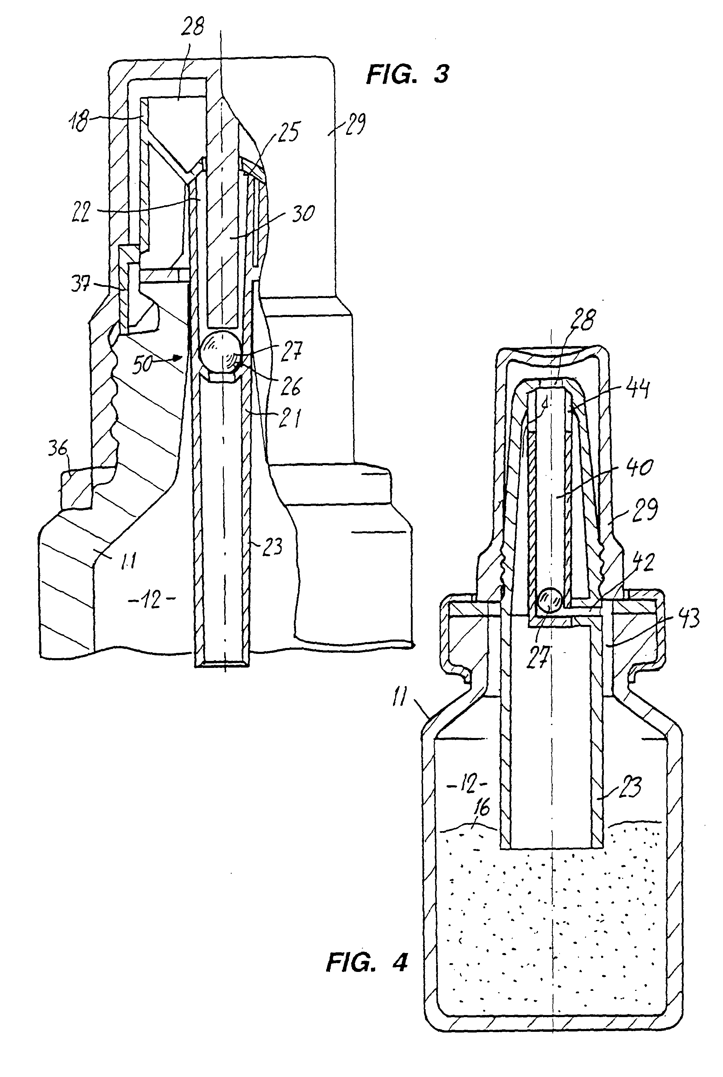

Fig. 3 zeigt in vergrößertem Maßstab eine Ausführung, bei der an der Kappe 29 ein mittig nach unten ragender, einen Vorsprung bildender Stift 30 angebracht ist, der durch die Öffnung des Ventilsitzes 25 und den größten Teil des erweiterten Austragkanales 22 ragt und so bemessen ist, daß im aufgeschraubten Zustand die untere Stirnfläche des Stiftes 30 den Ventilkörper 27 auf den unteren Ruheventilsitz 26 festlegt.

Ferner ist die Kappe 29 durch eine Originalitätssicherung 36 mit dem Behälter 11 verbunden. Sie enthält einen Ring, der über Sollbruchstellen mit der Kappe verbunden ist, die bei einer ersten Betätigung aufgerissen werden.

Ferner ist eine Kindersicherung 37 bekannter Bauart vorgesehen, die ein Abschrauben der Kappe 29 von dem Gehäuse 20 der Dosiereinrichtung 17 erst nach Durchführung einer bestimmten Betätigungsfolge, beispielsweise Niederdrücken der Kappe und danach Schraubbewegung, erlaubt.

Der Austragkanal 22 ist im Bereich der Ruheposition 50 nicht wesentlich weiter als der Ventilkörperdurchmesser, erweitert sich dann aber konisch. Dadurch ist eine sichere Beschleunigung des Ventilkörpers und ein langsamer Beginn des Austrags möglich. Zum Erzielen anderer Betriebsanforderungen kann auch eine andere Gestaltung des Austragkanales vorgesehen werden.

Das Mundstück 18 ist hier nicht, wie in Fig. 1 und 2, als ein Nasenadapter, sondern als ein in den Mund zu nehmendes Mundstück vorgesehen, durch das angesaugt werden kann. Im übrigen ist die Ausführung im wesentlichen baugleich mit der nach Fig. 1 und 2.

Das gilt auch für die Ausführung nach Fig. 4, die jedoch eine andere Art der Verschlußbetätigung zeigt. Der Verschluß 24a ist auch hier am oberen Ende des als Nasenadapter ausgebildeten Mundstückes 18 vorgesehen, und zwar in Form eines Ventilsitzes 25. Der als Kugel ausgebildete Verschlußkörper 27 ist am Ende eines Verschlußkörperkanales 40 vorgesehen, der in dem Gehäuse 20 zentrisch nach innen ragt, jedoch nicht dazu vorgesehen ist, von dem Medium durchströmt zu werden, jedenfalls nicht von der Hauptmenge. Der Verschlußkörper liegt am Ende dieses Verschlußkörperkanales 40 und ist an dessen Durchmesser weitgehend angepaßt, so daß er darin ähnlich wie ein Kolben bewegt werden kann. Am unteren Ende, d.h. an der Unterseite des Ventilkörpers 27, ist eine Verbindungsöffnung 41 vorgesehen, die im dargestellten Beispiel durch das in diesem Falle einen sehr großen Durchmesser aufweisende Saugrohr 23 hindurch bis in einen Ringspalt 43 ragt, der den Belüftungskanal 31 verlängert und an den Innenumfang des Flaschenhalses 23 angrenzt.

Im oberen Teil des Verschlußkörperkanales 40 sind in seiner im übrigen geschlossenen Außenwand Schlitze 44 vorgesehen, durch die das im Ringspalt 45 zwischen der Innenwandung des Gehäuses 20 und dem Verschlußkörperkanal 40 strömende Medium unter Benutzung des oberen Teiles des Verschlußkörperkanales zur Mündung 28 strömt.

### FUNKTION (FIG. 1 BIS 4)

Bei der Ausführung nach Fig. 1 wird zur Benutzung die Kappe 29 abgeschraubt und abgenommen. Dadurch wird der Austragskanal 22 über die Mündung 28 frei. Die Dosiereinrichtung kann nun an eine Körperöffnung angesetzt werden, beispielsweise mit dem Mundstück 18 in ein Nasenloch eingeführt werden.

Wenn der Benutzer nun durch das Nasenloch ansaugt, erzeugt er eine Strömung in dem Austragskanal und den daran anschließenden Saugrohr 23. Der in seiner Startposition 50 (Fig. 1) auf dem unteren Ruheventilsitz 26 liegende Ventilkörper 27 hebt von diesem ab und kann umströmt werden, da der Austragskanal 22 gegenüber dem Außendurchmesser des Ventilkörpers 27 erweitert ist. Durch das Saugrohr 23 wird Luft aus dem Behälter angesaugt und wirbelt im Falle der breiten trichterförmigen Ansaugöffnung 34 Pulver von der Oberfläche auf, das mit der Ansaugluft vermischt und somit als eine Luft/Pulver-Dispersion bzw. ein Festkörper-Aerosol in die entsprechende Körperhöhle des Benutzers eindringt und dort seine medizinische Wirkung erfüllen kann. Die Ausführung mit einem bis an den Boden des Behälters reichenden Saugrohr ist hauptsächlich bei solchen Pulvern vorgesehen, die selbst sehr locker sind und daher leicht in eine solche Aerosolform gebracht werden können.

Im Falle der Verwendung eines Belüftungsrohres 35 strömt die durch den Belüftungskanal 31 in den Behälter strömende, die abgesaugte Luft ersetzende Luft direkt unter die Oberfläche des Pulvers und sorgt für dessen gleichmäßige Verteilung in der Luft nach Art einer Aufwirbelung.

Nach dem Beginn der Ansaugung wirkt auf den Ventilkörper 27 eine Kraft ein, die durch Druckunterschiede oberhalb und unterhalb des Ventilkörpers erzeugt werden. Dieser Druckunterschied ist wiederum abhängig von der Drosselung in dem Umströmungsquerschnitt und damit von der Strömungsgeschwindigkeit. Der Ventilkörper bewegt sich also in dem Austragskanal aufwärts. Wenn er seine Bewegungsstrecke zwischen den beiden Ventilsitzen 25, 26 durchquert hat, legt er sich in der nun erreichten Schließposition 51 (Fig. 2) auf den oberen Ventilsitz 25 des Verschlusses 24 und schließt damit den Austragkanal für die Ansaugung ab.

Der Benutzer kann also nur soviel des Mediums (Luft/Feststoff-Dispersion) ansaugen, wie während der Zeit, in der sich der Ventilkörper aufwärts bewegt, an ihm vorbei durch den Austragskanal strömt. Da die Geschwindigkeit des Ventilkörpers von der Strömungsgeschwindigkeit bestimmt wird, bleibt die Dosiermenge relativ konstant, auch wenn unterschiedlich stark angesaugt wird. Bei höherer Ansauggeschwindigkeit ist die Menge je Zeiteinheit größer, dafür aber die Dosierzeit kürzer. Es ergibt sich also eine gewisse Dosierungsmengen-Automatik.

Die Funktion bei der Ausführung nach Fig. 3 ist entsprechend. Hier wird zum Abnehmen der Kappe zuerst das Aufbrechen der Originalitätssicherung und danach das Lösen der Kindersicherung erforderlich. Der Stift 30 gibt den bis dahin in der Startposition blockierten, den Ventilsitz 26 direkt verschließenden Ventilkörper frei. Die Ansaugung erfolgt in diesem Falle über den Mund, wozu das Mundstück eine entsprechende Gestalt hat.

Bei der Ausführung nach Fig. 4 umströmt das Medium den Verschlußkörperkanal 40 und strömt über die Schlitze 44 in den Verschlußkörperkanal und durch die Öffnung im Ventilsitz 25 hindurch. Dadurch wird im Verschlußkörperkanal 40 ein Unterdruck erzeugt, der den Ventilkörper 27 in diesem nach oben saugt. Dabei strömt über die Öffnung 42 aus dem Ringspalt 43 Luft nach, die aus dem Belüftungskanal 31 eindringt. Es ist jedoch auch möglich, die Öffnung 42 im Boden des hülsenförmigen Verschlußkörperkanales 40 vorzusehen, wodurch dann allerdings auch Dispersionen in diesen Kanal eindringt, wenn auch nur eine geringe Menge.

Nach Durchlaufen der Strecke zwischen der unteren Ruheposition des Ventilkörpers 27 und seiner oberen Verschlußposition am Ventilsitz 25 wird auch dort die Strömung unterbrochen und damit die Dosierung beendet.

Die die Dosierung bewirkende zeitliche Verzögerung wird also bei den dargestellten Beispielen durch die Zeit bestimmt, die der Ventilkörper benötigt, um die Verzögerungsstrecke zwischen seiner Ruheposition (bei Fig. 1 und 2 auf dem Ruheventilsitz und bei Fig. 3 auf dem Boden des Verschlußkörperkanales) zum Verschluß-Ventilsitz 25 zu laufen. Diese Zeit könnte allerdings auch anders bestimmt, vergleichmäßigt oder weiter verzögert werden, beispielsweise durch mechanische oder andere Mittel. Man könnte beispielsweise daran denken, den Ventilkörper auf einem Steilgewinde laufen zu lassen, das zusätzlich auch eine Drehbewegung ausführt, um die Dosierzeit zu verlängern. Auch andere Antriebsmittel für den Ventilkörper 25 sind möglich. Der Ventilkörper selbst ist zwar vorzugsweise eine Kugel, da diese am einfachsten und unproblematischsten in Herstellung und Anwendung ist, es könnte jedoch auch eine andere Ventilkörperform Anwendung finden, beispielsweise ein Ventilkörper mit einem mittleren Stift und zwei kegelförmigen oder kugeligen Enden, der durch eine Öffnung ragt und dessen Enden mit an beiden Seiten der Öffnung angeordneten Ventilsitzen zusammenarbeiten. Dabei bestimmt die Länge des Stiftes die Verzögerungsstrecke.

Die beschriebene Dosiereinrichtung ist besonders vorteilhaft für die Anwendung bei Pulver/Luft-Dispersionen, und dort bevorzugt bei solchen Einrichtungen, die mit Ansaugung von außen her arbeiten. Bei diesen Einrichtungen ist das Dosieren ein besonderes Problem, weil keine in der Dosiereinrichtung selbst vorgesehene Fördereinrichtung für das Medium, wie beispielsweise eine Pumpe, vorgesehen ist. Es ist jedoch möglich, die Dosiereinrichtung bei anderen Vorrichtungen, beispielsweise einem Flüssigkeitszerstäuber nachgeschaltet, einzusetzen. Dies kann beispielsweise bei einer Vorrichtung wichtig sein, die mit einem Druckgasbehälter arbeitet.

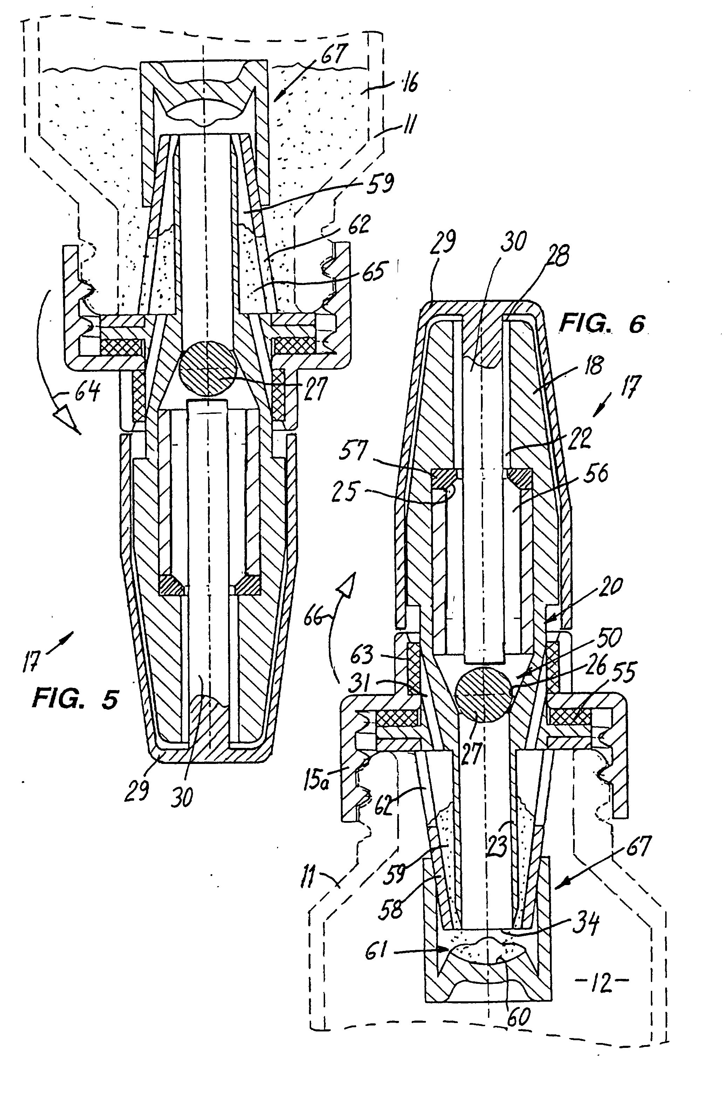

### BESCHREIBUNG DES AUSFÜHRUNGSBEISPIELS (FIG. 5 BIS 8)

Der Grundaufbau der Dosiereinrichtung nach Figuren 5 bis 8 ist entsprechend dem nach Fig. 1 und 2. Auf den Behälter 11 ist das Gehäuse 20 der Dosiereinrichtung 17 mittels einer Überwurfmutter 15a aufgeschraubt. Das Gehäuse 20 ist zu einem Mundstück 18 geformt, in dessen Inneren ein Austragkanal 22 zu einer Mündung 28 führt.

In einem Abschnitt 56 des Austragkanals 22 mit vergrößertem Querschnitt ist der Ventilkörper 27 in Form einer Stahlkugel längs beweglich angeordnet. Seine beiden Endlagen werden durch die Ventilsitze 25 und 26 bestimmt.

Der Ventilsitz 25 befindet sich an einem Ventilsitzring 57, der aus einem Kunststoffmaterial mit magnetischen Eigenschaften besteht. Dazu können an sich bekannten Kunststoffe mit eingelagerten Magnetpartikeln verwendet werden.

Vom Gehäuse 20 ragt in den Behälter hinein ein Saugrohr 23, das einseitig an den Ventilsitz 26 anschließt und auf seiner Unterseite eine Mündung 34 hat, die von einem konischen Rohrabschnitt 58 umgeben ist. Der sich zwischen Saugrohr 23 und Rohrabschnitt 58 bildende Ringkanal 59 verengt sich konisch zur Mündung 34 hin und ist auf einen schalenförmigen Boden 60 einer Mediumsvorlage 61 gerichtet, auf der sich das pulverförmige Medium 16 vor der Zerstäubung bzw. Mitnahme durch die Luft sammelt. Der Rohrabschnitt 58 ist in seinem in Fig. 6 oberen Teil mit Durchbrüchen 62 versehen, durch die der Ringkanal 59 mit dem Behälterinneren 12 verbunden ist.

Die Schraubkappe 15a und das Gehäuse 20 weisen am Umfang mehrere Belüftungskanäle 31 auf, die das Behälterinnere 12 mit der Außenluft verbinden, jedoch unter Zwischenschaltung eines Feinfilters 63, der wie ein Ring um die äußeren Mündungen der Belüftungsöffnungen herumliegt und eine Kontamination des Mediums durch Eindringen von Keimem von außen her verhindert.

Die Kappe 29 hat, wie bei Fig. 3, einen mittleren stiftförmigen Vorsprung 30, der durch den gesamten Austragkanal 22 bis nahe zum Ventilsitz 26 reicht.

### FUNKTION (FIG. 5 BIS 8)

Die dargestellte Dosiereinrichtung 17 ist zur Betätigung mittels Ansaugung über Mund oder Nase eines Patienten vorgesehen. Sie könnte jedoch eine (nicht dargestellte) Luftpumpe, z.B. im Behälterbereich, besitzen, die den Austrag mittels eines fremderzeugten Luftstromes bewirkt.

Im Ruhezustand nimmt die Dosiervorrichtung die in Fig. 6 gezeigte Lage ein. Die Kappe 29 ist aufgesetzt und der Stift 30 hält die Ventilkugel 27 auf dem Ventilsitz 26, d.h. in der Startposition 50. Zu Beginn der Betätigung wird die Dosiereinrichtung 17 durch Kippen um 180° (siehe Pfeil 64) in die in Fig. 5 gezeigte Überkopf-Lage gedreht. Dadurch fällt das Medium 16 in den Behälterhals und füllt die sich im oberen Teil des Ringkanals 59 angrenzend an die Durchbrüche 62 bildende Sammelkammer 65. Nach oben ist die Befüllung der Sammelkammer durch den normalen Schüttkegel des Mediums (Pulvers) begrenzt. Die Ventilkugel 27 auf dem Ventilsitz 26 verhindert ein Herausfallen von eventuell noch in der Vorkammer 67 vorhandenem Medium 16.

Danach wird die Dosiereinrichtung wieder in die aufrechte Lage (Fig. 6) gedreht (Pfeil 66). Das Medium 16 fällt wieder in den Behälter zurück, bis auf den Teil, der von der Sammelkammer 65 in den konischen Ringkanal 59 fällt und von dort in die Vorkammer 67 gelangt, deren schalenförmiger Boden 60 die Mediumsvorlage 61 bildet.

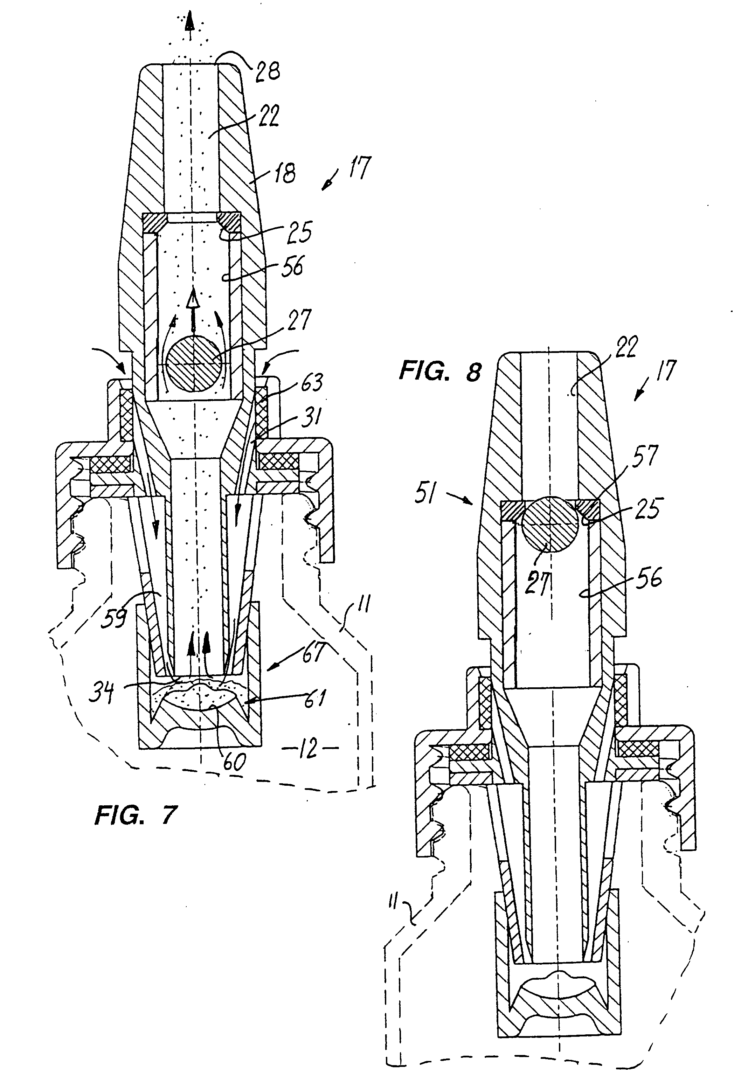

Wenn jetzt durch Ansaugen am Mundstück 18 (Fig. 7) die Ausgabefunktion eingeleitet wird, dann öffnet sich unter dem entstehenden Sog das untere Ventil, d.h. die Ventilkugel 27 hebt vom Ventilsitz 26 ab und die Kugel bewegt sich aufgrund des Widerstandes des vorbeiströmenden Medium/Luftgemisches entgegen der Schwerkraft im Abschnitt 56 des Austragkanals 22 nach oben. Dabei strömt Luft über den Filter 63 und die Belüftungsöffnungen 31 ins Behälterinnere und über den Ringkanal 59 in die Vorkammer 67. Infolge der Ringdüsenanordnung der Mündung des Ringkanals 59, die die Saugrohrmündung 34 umgibt, wirbelt der eintretende Luftstrom das auf der Mediumsvorlage 61 liegende Medium auf und trägt es als gleichmäßiges Feststoff/Luftgemisch über das Saugrohr und den Austragskanal 22 aus. Es ist somit eine sehr gute und gleichmäßige Aerosolbildung möglich und die Ansammlung einer bestimmten Mediumsmenge in der Vorkammer sorgt für eine Vordosierung, die gewollte oder ungewollte Manipulationsmöglichkeiten der Bedienung ausschließt und für eine stets ausreichend gleichmäßige Dosierung sorgt.

Wenn die Kugel 27 unter der Wirkung des Luftstromes den Ventilsitz 25 (Schließposition) erreicht, wird schlagartig die Ausgabe unterbrochen (Fig. 8). Die Kugel bleibt auch nach Aufhebung des Sogs oder Innendrucks an dem magnetischen Ring 57 infolge der Magnetwirkung hängen. Es wird somit verhindert, daß der Patient nochmals ansaugt, wenn die Kugel evtl. wieder nach unten gefallen sein sollte. Durch diese Magnetwirkung wird also beim Ausführungsbeispiel ein Haltemittel gebildet, das aber auch aus mechanischen oder anderen Haltern bestehen könnte, beispielsweise in den Strömungskanal ragenden Kunststofflappen o. dgl.

Der Patient muß nun erst wieder die Kappe 29 aufstecken, damit der Stift 30 die Kugel vom Ventilsitz 25 löst und sie wieder in die Ruhe- oder Startposition 51 bringt. Danach könnte ein weiterer Betätigungszyklus erfolgen.

Die Ausführungsform nach den Figuren 5 bis 8 erlaubt also eine besonders zuverlässige und gleichmäßige Zerstäubung mit Vordosierung und sichert die Dosiereinrichtung gegen versehentliche Doppelbetätigung. Außerdem ermöglicht sie eine keimfreie Lagerung des Pulvers auch zwischen einzelnen Betätigungsvorgängen.

## Patentansprüche

1. Dosiereinrichtung für strömungsfähige Medien, insbesondere in Austragsvorrichtungen für pharmazeutische oder kosmetische Produkte, wie Pulver/Luft-Dispersionen,
1.1 wobei das Medium einen Austragkanal (22) durchströmt,
1.2 mit einem von einer Startposition (50) in eine Endposition über eine Wegstrecke begrenzt beweglichen Ventilkörper (27),
**dadurch gekennzeichnet**,
1.3 dass in dem Austragkanal (22) ein den Ventilkörper umgebender Umströmungsquerschnitt (45) vorgesehen ist,
1.4 dass der entgegen der Strömungsrichtung durch eine Kraft, wie der Schwerkraft, belastete Ventilkörper (27) Teil eines Endverschlusses (24) ist,
1.4.1 in einer Schliessposition (51) mit einem Verschluss-Ventilsitz (25) zusammenwirkt,
1.4.2 und im Austragkanal (22) durch den Widerstand des ihn in dem Umströmungsquerschnitt (45) umströmenden Mediums unter der Wirkung der Strömung entgegen der Kraft von einer Startposition (50) zeitverzögert in eine Schließposition (51) mitgenommen wird.

2. Dosiereinrichtung für strömungsfähige Medien, insbesondere in Austragsvorrichtungen für pharmazeutische oder kosmetische Produkte, wie Pulver/Luft-Dispersionen,
2.1 wobei das Medium einen Austragkanal (22) durchströmt,
2.2 mit einem von einer Startposition (50) in eine Endposition über eine Wegstrecke begrenzt beweglichen Ventilkörper (27),
**dadurch gekennzeichnet**,
2.3 dass in dem Austragkanal (22) ein den Ventilkörper umgehender Umströmungsquerschnitt (45) vorgesehen ist,
2.4 dass der entgegen der Strömungsrichtung durch eine Kraft, wie der Schwerkraft, belastete Ventilkörper (27) Teil eines Endverschlusses (24) ist,
2.4.1 der in einer Schliessposition (51) mit einem Verschluss-Ventilsitz (25) zusammenwirkt,
2.4.2 und im Austragkanal (22) durch den von der Strömung im Austragkanal (22) erzeugten Unterdruck entgegen der Kraft aus einer außerhalb des Austragkanales (22) liegenden Startposition (50) zeitverzögert in die Schließposition (51) bewegbar ist.

3. Dosiereinrichtung nach Anspruch 1 oder 2, **dadurch gekennzeichnet, daß** der Ventilkörper (27) eine Kugel ist.

4. Dosiereinrichtung nach einem der vorhergehenden Ansprüche, **dadurch gekennzeichnet, daß** der Ventilkörper (27) in einem ihm gegenüber erweiterten Austragkanal (22) angeordnet ist.

5. Dosiereinrichtung nach einem der vorhergehenden Ansprüche, **dadurch gekennzeichnet, daß** der Ventilkörper (27) in seiner Startposition (50) einen Ruheverschluß für den Austragkanal (22) bewirkt, durch Auflage auf einem Ventilsitz (26) des Ruheverschlusses oder durch Haltemittel (57) für den Verschluß (24), die diesen in der Schließposition (51) halten, bis er durch mechanische Öffnungsmittel (30) wieder geöffnet wird, wobei vorzugsweise die Haltemittel ein magnetisches Bauteil beinhalten und/oder die Öffnungsmittel einen an einer Verschlußkappe vorgesehenen Vorsprung (30) aufweisen.

6. Dosiereinrichtung nach einem der vorhergehenden Ansprüche, **dadurch gekennzeichnet**, **dadurch gekennzeichnet, daß** ein sich zwischen dem Ventilkörper (25) und dem Austragkanal (22) bildender Umströmungsquerschnitt (45) über die zwischen Start- und Schließposition (50, 51) liegende Länge des Austragkanales (22) veränderlich ist.

7. Dosiereinrichtung nach einem der Ansprüche 2 bis 6, **dadurch gekennzeichnet, daß** der Ventilkörper (25) in einem ggf. belüfteten, nicht von der Hauptmenge des Mediums über seine ganze Länge durchströmten Verschlußkörperkanal (40) angeordnet ist.

8. Dosiereinrichtung nach einem der vorhergehenden Ansprüche, **dadurch gekennzeichnet, daß** sie in einem Inhalator für Pulver/Luft-Dispersion angeordnet ist und vorzugsweise ein Mundstück (18) zum Ansaugen der Dispersion aus einem Pulverbehälter (11) enthält, wobei der Austragkanal (22) zwischen einer in den Behälter (11) mündenden Ansaugöffnung (34) und dem Mundstück (18) angeordnet ist und daß wenigstens ein Belüftungskanal (31) für den Behälter (11) vorgesehen ist.

9. Dosiereinrichtung nach einem der vorhergehenden Ansprüche, **dadurch gekennzeichnet, daß** eine Verschlußkappe (29) vorgesehen ist, die Mündungen (28) des Austragkanales (22) und ggf. eines Belüftungskanales (31) überdeckt und vorzugsweise einen in den Austragkanal (22) ragenden Vorsprung (30) aufweist, der einen Ventilkörper (27) des Verschlusses (24) auf einem Ruheverschluß-Ventilsitz (26) festlegt.

10. Dosiereinrichtung nach einem der vorhergehenden Ansprüche, daß zur Unterstützung des Austragens einer Pulver/Luft-Dispersion aus einem Pulver (16) enthaltenden Behälter (11), in dem die Pulver/Luft-Dispersion erzeugt wird, eine in den Behälter (11) führende Belüftungsöffnung (31, 35) in oder auf den Pulvervorrat (16) in dem Behälter (11) gerichtet ist, so daß die infolge des Austrags nachgesaugte Luft das Pulver (16) aufwirbelt.

11. Dosiereinrichtung nach einem der vorhergehenden Ansprüche, **dadurch gekennzeichnet, daß** im Medienfluß der Startposition (50) eine Vorkammer (67) vorgeschaltet ist, die eine durch Kippen der Dosiereinrichtung (17) befüllbare Vordosiereinrichtung und/oder eine Mediumsvorlage (61) vor der Eingangsöffnung (34) eines Saugrohres (23) bildet.

12. Dosiereinrichtung nach Anspruch 11, **dadurch gekennzeichnet, daß** die Vorkammer (67) einen die Mediumsvorlage (61) bildenden, ggf. schalenförmig gewölbten, Boden (60) und/oder einen vorzugsweise ringkanalförmigen, sich verengenden Einlaßkanal (59) aufweist, dessen Mündung bevorzugt auf die Mediumsvorlage (61) gerichtet ist, und dem eine Sammelkammer (65) vorgeschaltet ist, in der sich beim Kippen der Dosiereinrichtung (Pfeile 64, 66) eine Dosis des Mediums (16) sammelt.

## Claims

1. Dosing device for flowable media, particularly in discharge apparatuses for pharmaceutical or cosmetic products, such as powder/air dispersions,
1.1 in which the medium flows through a discharge channel (22),
1.2 with a valve body movable in limited manner over a path length from a starting position (50) into an end position,
**characterized in that**
1.3 in the discharge channel (22) is provided a flow-round cross-section (45) surrounding the valve body,
1.4 the valve body (27) loaded counter to the flow direction by a force, such as the force of gravity, is part of an end closure (24), which
1.4.1 in a closed position (51) cooperates with a closure valve seat (25),
1.4.2 and in the discharge channel (22) is moved by the resistance of the medium flowing round it in the flow-round cross-section (45) in time-delayed manner from a starting position (50) into a closed position (51) under the action of the flow and counter to the force.

2. Dosing device for flowable media, particularly in discharge apparatuses for pharmaceutical or cosmetic products, such as powder/air dispersions, 2.1 in which the medium flows through a discharge channel (22),
2.2 with a valve body (27) movable in limited manner over a path length from a starting position (50) into an end position,
**characterized in that**
2.3 in the discharge channel (22) a flow-round cross-section (45) surrounding the valve body is provided,
2.4 the valve body (27) loaded counter to the flow direction by a force, such as the force of gravity, is part of an end closure (24),
2.4.1 which cooperates in a closed position (51) with a closure valve seat (25),
2.4.2 and in the discharge channel (22) is movable in time-delayed manner from a starting position (50) located outside the discharge channel (22) into the closed position (51) through the underpressure produced by the flow in said discharge channel (22) and counter to the force.

3. Dosing device according to claim 1 or 2, **characterized in that** the valve body (27) is a ball.

4. Dosing device according to one of the preceding claims, **characterized in that** the valve body (27) is located in a discharge channel (22) widened compared therewith.

5. Dosing device according to one of the preceding claims, **characterized in that** in its starting position (50) the valve body (27) brings about a rest closure for the discharge channel (22) by engaging on a valve seat (26) of the rest closure or by retaining means (57) for the closure (24), which maintain the latter in the closed position (51) until it is reopened by mechanical opening means (30), in which preferably the retaining means incorporate a magnetic component and/or the opening means have a projection (30) located on a closure cap.

6. Dosing device according to one of the preceding claims, **characterized in that** a flow-round cross-section (45) formed between the valve body (25) and the discharge channel (22) is variable over the length of the discharge channel (22) located between the starting and closure positions (50, 51).

7. Dosing device according to one of the claims 2 to 6, **characterized in that** the valve body (25) is located in an optionally vented closure body channel (40) through which the main quantity of the medium does not flow over its entire length.

8. Dosing device according to one of the preceding claims, **characterized in that** it is located in an inhaler for powder/air dispersion and preferably contains a mouthpiece (18) for sucking the dispersion out of a powder container (11), the discharge channel (22) being located between a suction opening (34) issuing into the container (11) and the mouthpiece (18) and that at least one ventilation channel (31) is provided for the container (11).

9. Dosing device according to one of the preceding claims, **characterized in that** a closure cap (29) is provided, which covers apertures (28) of the discharge channel (22) and optionally a ventilation channel (31) and preferably has a projection projecting into the discharge channel (22) and which fixes a valve body (27) of the closure (24) on a rest closure valve seat (26).

10. Dosing device according to one of the preceding claims, **characterized in that** for aiding the discharge of a powder/air dispersion from a container (11) containing a powder (16) and in which the powder/air dispersion is produced, a ventilation opening (31, 35) leading into the container (11) is directed into or onto the powder supply (16) in the container (11), so that the air sucked in due to the discharge brings about a turbulence of the powder (16).

11. Dosing device according to one of the preceding claims, **characterized in that** a prechamber (67) is located in the medium flow upstream of the starting position (50) and forms a predosing device fillable by tilting the dosing device (17) and/or a medium supply (61) upstream of the inlet port (34) of a suction tube (23).

12. Dosing device according to claim 11, **characterized in that** the prechamber (67) has a base (60), optionally arched in shell-like manner and forming the medium supply (61) and/or a preferably ring duct-like, narrowing inlet channel (59), whose opening is preferably directed onto the medium supply (61) and upstream of which is positioned a collecting chamber (65) in which is collected a dose of the medium (16) on tilting the dosing device (arrows 64, 66).

## Revendications

1. Moyen de dosage pour matières capables d'écoulement, notamment dans des dispositifs de distribution pour produits pharmaceutiques ou cosmétiques, telles que des dispersions poudre/air,
1.1 la matière s'écoulant à travers un conduit de décharge (22),
1.2 avec un corps de soupape (27) mobile entre les limites d'un certain parcours à partir d'une position initiale (50) jusqu'à une position terminale,
**caractérisé**
1.3 en ce que dans le conduit de décharge (22) est prévue une section de contoumement (45) entourant le corps de soupape,
1.4 en ce que le corps de soupape (27), chargé par une force, telle que la force de gravité, en direction opposée à la direction d'écoulement, fait partie d'un moyen de fermeture terminal (24),
1.4.1 qui agit dans une position de fermeture (51) ensemble avec un siège de soupape de fermeture (25)
1.4.2 et qui est entraîné dans le conduit de décharge (22) par la résistance de la matière qui s'écoule autour de lui dans la section de contournement (45), sous l'action de l'écoulement contre la force à partir d'une position initiale (50) dans une position de fermeture (51) avec un certain retard.

2. Moyen de dosage pour des matières capables de s'écouler, notamment dans des dispositifs de distribution pour produits phamaceutiques ou cosmétiques, telles que des dispersions poudre/air,
2.1 la matière s'écoulant à travers un conduit de décharge (22),
2.2 avec un corps de soupape (27) mobile entre les limites d'un certain parcours à partir d'une position initiale (50) jusqu'à une position terminale,
**caractérisé**
2.3 en ce que dans le conduit de décharge (22) est prévue une section de contournement (45) entourant le corps de soupape,
2.4 en ce que le corps de soupape (27), chargé par une force, telle que la force de gravité, en direction opposée à la direction d'écoulement, fait partie d'un moyen de fermeture terminal (24),
2.4.1 qui agit dans une position de fermeture (51) ensemble avec un siège de soupape de fermeture (25)
2.4.2 et qui peut être déplacé dans le conduit de décharge (22) par la sous-pression produite par l'écoulement dans le conduit de décharge (22) contre une force, à partir d'une position initiale (50) située à l'extérieur du conduit de décharge (22) de manière retardée jusqu'à la position terminale (51).

3. Moyen de dosage d'après la revendication 1 ou 2, **caractérisé en ce que** le corps de soupape (27) est une bille.

4. Moyen de dosage d'après une des revendications précédentes, **caractérisé en ce que** le corps de soupape (27) est disposé dans un conduit de décharge (22) plus large par rapport à lui.

5. Moyen de dosage d'après une des revendications précédentes, **caractérisé en ce que** le corps de soupape (27) dans sa position initiale (50) produit une fermeture au repos pour le conduit de décharge (22), par placement sur un siège de soupape (26) de la fermeture au repos ou bien par des moyens de retenue (57) pour le moyen de fermeture (24) qui le gardent dans la position de fermeture (51), jusqu'à ce qu'il soit ouvert à nouveau par des moyens d'ouverture (30) mécaniques, les moyens de retenue comprenant de préférence une pièce magnétique et/ou les moyens d'ouverture présentant une saillie (30) prévue sur un capuchon de fermeture.

6. Moyen de dosage d'après une des revendications précédentes, **caractérisé en ce qu'**une section de contournement (45) qui se forme entre le corps de soupape (25) et le conduit de décharge (22) est variable sur la longueur du conduit de décharge situé entre la position initiale et terminale (50, 51).

7. Moyen de dosage d'après une des revendications de 2 à 6, **caractérisé en ce que** le corps de soupape (25) est disposé dans un conduit de corps d'occlusion (40) éventuellement aéré, et qui n'est pas traversé sur toute sa longueur par l'écoulement de la masse principale de matière.

8. Moyen de dosage d'après une des revendications précédentes, **caractérisé en ce qu'**il est disposé dans un inhalateur pour dispersions poudre/air et qu'il comprend de préférence une embouchure (18) pour aspirer la dispersion à partir d'un réservoir de poudre (11), le conduit de décharge (22) étant disposé entre une ouverture d'aspiration (34) débouchant dans le réservoir (11) et l'embouchure (18) et **en ce qu'**au moins un conduit d'aération (31) est prévu pour le réservoir (11).

9. Moyen de dosage d'après une des revendications précédentes, **caractérisé en ce qu'**un chapeau-obturateur (29) est prévu qui recouvre les orifices (28) du conduit de décharge (22) et éventuellement d'un conduit d'aération (31) et qui présente de préférence une saillie (30) saillant dans le conduit de décharge (22) laquelle fixe un corps de soupape (27) du moyen de fermeture (24) sur un siège de soupape de moyen de fermeture au repos (26).

10. Moyen de dosage d'après une des revendications précédentes, **caractérisé en ce qu'**en tant que soutien pour l'action de distribution d'une dispersion poudre/air à partir d'un réservoir (11) renfermant de la poudre (16) et dans lequel est produite la dispersion poudre/air, une ouverture d'aération (31, 35) menant dans le réservoir (11) est dirigée dans ou sur la réserve de poudre (16) dans le réservoir (11), de sorte que l'air aspiré à la suite de l'action de décharge soulève des tourbillons de poudre (16).

11. Moyen de dosage d'après une des revendications précédentes, **caractérisé en ce que** dans le cours de l'écoulement de matière en amont de la position initiale (50) est placée de manière fonctionnelle une préchambre (67), qui constitue un moyen de prédosage pouvant être chargée par basculage du moyen de dosage (17), et/ou un moyen de mise en position de matière (61) devant l'ouverture d'entrée (34) d'un tuyau d'aspiration (23).

12. Moyen de dosage d'après la revendication 11, **caractérisé en ce que** la préchambre (67) présente un fond (60), éventuellement bombé en forme de cuvette et formant le moyen de mise en position de matière (61) et/ou un conduit d'admission (59) de préférence en forme de conduit annulaire et convergent, dont l'embouchure est orientée de préférence sur le moyen de mise en position de matière (61), et en amont duquel se trouve de manière fonctionnelle une chambre de captage (65) dans laquelle s'accumule une dose de matière (16) pendant le basculage du moyen de dosage (flèches 64, 66).
